**Europäisches Patentamt**

**European Patent Office**

**Office européen des brevets**

(19)

(11) Veröffentlichungsnummer : **0 046 926
B1**

(12) # EUROPÄISCHE PATENTSCHRIFT

(45) Veröffentlichungstag der Patentschrift :
**11.12.85**

(51) Int. Cl.⁴ : **A 61 F  2/38**

(21) Anmeldenummer : **81106426.0**

(22) Anmeldetag : **19.08.81**

(54) **Kniegelenk-Endoprothese.**

(30) Priorität : **03.09.80 DE 3033081**

(43) Veröffentlichungstag der Anmeldung :
**10.03.82 Patentblatt 82/10**

(45) Bekanntmachung des Hinweises auf die Patenterteilung : **11.12.85 Patentblatt 85/50**

(84) Benannte Vertragsstaaten :
**CH DE FR GB LI**

(56) Entgegenhaltungen :
**DE-A- 1 906 284
DE-A- 2 142 820
DE-A- 2 823 215
DE-B- 2 728 690
FR-A- 2 266 492
FR-A- 2 290 880
US-A- 3 064 645**

(73) Patentinhaber : **Waldemar Link GmbH & Co
Barkhausenweg 10
D-2000 Hamburg 63 (DE)**

(72) Erfinder : **Keller, Arnold
An der Naher Furth 5
D-2061 Kalhude (DE)**

(74) Vertreter : **Glawe, Delfs, Moll & Partner Patentanwälte
Rothenbaumchaussee 58 Postfach 2570
D-2000 Hamburg 13 (DE)**

**Beschreibung**

Die Erfindung bezieht sich auf eine Kniegelenk-Endoprothese mit einem mindestens eine Gelenkgleitfläche und eine Verankerungsfläche bildenden Prothesenteil der aus zwei Einzelteilen besteht, von denen einer die Gelenkgleitfläche und der andere die Veranerkungsfläche trägt und die unter Zwischenlage von elastischem Dämpfungsmaterial miteinander zur Übertragung von Druck-, Schub- und Torsionskräften verbunden sind (FR-A-2 266 492).

Gelenkendoprothesen sind überwiegend aus einem verhältnismäßig harten und unelastischen Material hergestellt, damit sie einerseits mit ihren Verankerungsflächen dauerhaft im Knochen verankert werden können und damit sie andererseits an den Gleitflächen die Gelenkkräfte sicher übertragen können. Natürliche Gelenke sind demgegenüber wesentlich nachgiebiger. Diese verminderte Abpufferung der an Endoprothesen auftretenden Kräfte hat einen ungünstigen Einfluß auf die Haltbarkeit ihrer Verankerung und auf den Abrieb der Gleitflächen. Dies gilt insbesondere dann, wenn die natürliche Stützung durch den jeweiligen Bandapparat des Patienten eingeschränkt ist oder fehlt. Dann muß die Prothese nicht nur den Bewegungsablauf nachvollziehen, sondern auch alleine in der Lage sein, die auftretenden Knick-, Torsions- und Biegekräfte aufzufangen. Erschwert wird dies noch durch den Umstand, daß der Bewegungsablauf eines natürlichen Gelenkes durch eine Prothese nur unvollkommen nachvollzogen werden kann. Das gilt besonders wenn man bedenkt, daß von einem zum andern Patienten ganz unterschiedliche, individuelle Verhältnisse vorhanden sind. Beispielsweise ist es oft unvermeidlich, daß der Dreh- und Gleitpunkt bei der prothetischen Versorgung gegenüber den optimalen Verhältnissen des ursprünglichen Gelenks verschoben wird, wodurch die Lastaufnahme und Kraftübertragung ungünstig verlagert wird. Die Verwendung von Prothesenteilen aus einem Material, das einen Elastizitätsmodul ähnlich dem des Knochengelenks hat (DE-A 2 142 820), kann die erforderliche Elastizität der gesamten Prothese nicht gewährleisten, weil stets einige Teile derselben aus mechanischen Gründen aus hartem Material bestehen müssen. Außerdem kommt in den weitaus meisten Fällen die Verwendung solchen Materials mit einem dem Knochen ähnlichen Elastizitätsmodul mangels Bewährung nicht in Frage.

Zur Vermeidung dieser Nachteile hat man bei der Kniegelenk-Endoprothese der eingangs genannten Art zwischen einem die Gelenkfläche bildenden, austauschbaren Plateau und dem tibiaseitig zu verankernden Prothesenteil eine elastische Schicht vorgesehen, die die Druckkräfte zwischen dem Plateau und dem Verankerungsteil überträgt. Hingegen werden Schub- und Torsionskräfte unmittelbar von dem Plateau auf den Verankerungsteil übertragen mittels an dem Plateau angebrachten seitlichen Kufen, die den Verankerungsteil umgreifen. — Diese Anordnung ist nachteilig, weil die Flanken des Verankerungsteils freiliegen müssen, damit die Kufen des Plateaus daran angreifen können. Damit die Kufen des Plateaus, das im Hinblick auf einen niedrigen Reibungskoeffizienten aus synthetischem Material wie Polyäthylen besteht, die zur Verklammerung an dem Verankerungsteil notwendige, kräftige Dauerelastizität besitzen, muß das Plateau mit metallischen Verstärkungseinlagen versehen werden, so daß nur eine vergleichsweise dünne Verschleißschicht aus dem synthetischen Material zur Verfügung steht, so daß in ungünstigen Fällen schon relativ frühzeitig nachoperiert werden muß. Außerdem besteht bei dem Vorhandensein metallischer Teile im Plateau die Gefahr, daß bei vollständigem Abrieb der synthetischen Deckschicht diese metallischen Teile unmittelbar an den Gleitkufen des oberen Prothesenteils reiben und diese daher ebenfalls ersetzt werden müssen. — Schließlich hat die bekannte Ausführung den Nachteil, daß Schub- und Torsionskräfte nicht hinreichend abgefedert werden können, weil das Plateau zur Aufbringung einer hinreichenden Verklammerungskraft ziemlich steif ausgeführt werden muß. Die Erfahrung hat jedoch gezeigt, daß gerade bei Kniegelenk-Endoprothesen oftmals beträchtliche Torsionsbeanspruchung auch in stoßartiger Form auftreten kann.

Ferner bekannt ist eine Kniegelenk-Endoprothese (DE-B 2 728 690), bei der die Verbindung zwischen dem Verankerungsteil und dem die Gelenkfläche bildenden Prothesenteil über einen metallischen Zapfen vermittelt wird, der in eine Polyäthylen-Buchse eingreift. Diese ist zwar elastischer als Metall, kann aber dennoch eine hinreichende Abfederung nicht gewährleisten. Die elastischen Eigenschaften der Buchse sollen bei der bekannten Prothese vielmehr lediglich dazu dienen, einen hinreichen Paßsitz auch bei Kipp- und Pendelbewegungen zu gewährleisten. Eine Übertragung von Torsionskräften ist nicht vorgesehen; diese bleiben dem Bandapparat überlassen.

Bei einer bekannten Hüftgelenk-Endoprothese (US-PS 3 064 645) ist der die kugelige Gelenkfläche bildende Prothesenkopf mit dem ihn tragenden Teller am Ende des Prothesenschafts einerseits über elastisches Dämpfungsmaterial und andererseits gleitbar über einen an dem Teller angebrachten, zylindrischen Kragen verbunden. Dies ermöglicht der Gelenkkugel eine gedämpfte Relativbewegung gegenüber dem Schaft in Längsrichtung, d. h. in Richtung der Längsachse des zylindrischen Kragens, während diejenigen Kräfte, die quer zu dieser Achse verlaufen, unmittelbar von der Gelenkkugel auf den Kragen übertragen werden. Nur Längskräfte sowie Torsionskräfte, auf die es bei einem Kugelgelenk ohnehin weniger ankommt, werden daher durch die bekannte Anordnung gedämpft, während die Schubkräfte ungedämpft bleiben.

Der Erfindung liegt die Aufgabe zugrunde, die Kniegelenk-Endoprothese der eingangs genannten Art in der Weise zu verbessern, daß Stöße jeglicher Richtung gedämpft übertragen werden können.

Die erfindungsgemäße Lösung besteht darin, daß die Verbindung zwischen dem die Gelenkgleitfläche bildenden Einzelteil und dem die Verankerungsfläche bildenden Einzelteil der Prothese ausschießlich aus dem elastischen Dämpfungsmaterial besteht.

Zweckmäßigerweise erstreckt sich das elastische Dämpfungsmaterial im wesentlichen senkrecht zur vorherrschenden Kraftrichtung, damit es überwiegend auf Druck bzw. Zug belastet wird. In manchen Fällen kann es jedoch auch wünschenswert sein, eine gewisse Scherwirkung auszuüben ; in diesem Falle kann es sich schräg zur vorherrschenden Kraftrichtung erstrecken. Zweckmäßig ist plattenförmige Verteilung des elastischen Materials. Es kann aber auch ringförmig insbesondere am Rand der einander zugewendeten Flächen der von dem elastischen Dämpfungsmaterial getrennten Einzelteile der Prothese angeordnet sein.

Nach einem weiteren Merkmal der Erfindung kann das elastische Dämpfungsmaterial aus einer Vielzahl elastischer Körper bestehen, die an einander gegenüberstehenden Flächen befestigt sind. Diese Körper sind vorzugsweise zylinder-, kugel- oder quaderförmig.

In vielen Fällen wird man vorsehen, daß die Elastizität des elastischen Dämpfungsmaterials in dem von diesem eingenommenen Bereich konstant ist. Häufig wird jedoch die Gelenkverbindung auf einer Seite mehr belastet als auf der anderen. Dem kann man dadurch Rechnung tragen, daß man im höher belasteten Teil eine geringere Elastizität des elastischen Dämpfungsmaterials vorsieht. Je nach Anwendungszweck kann zweckmäßigerweise vorgesehen werden, daß die Elastizität über den von dem elastischen Dämpfungsmaterial eingenommen Material eingenommenen Bereich variiert. Besteht es aus einer Vielzahl elastischer Körper, so kann man dies beispielsweise dadurch erreichen, daß elastische Körper unterschiedlicher Größe vorgesehen werden.

Das elastische Dämpfungsmaterial muß physiologisch einwandfrei sein, z. B. aus Silikon-Gummi bestehen.

Die Erfindung wird im folgenden beispielsweise anhand von vorteilhaften Ausführungsformen unter Bezugnahme auf die beigefügte Zeichnung beschrieben. Es zeigen :

Figur 1 in einer schematischen Ansicht eine Darstellung der ungleichmäßigen Belastung der Gleitflächen einer Kniegelenk-Endoprothese ;

Figur 2 teilweise im Querschnitt eine Ansicht einer Ausführungsform der erfindungsgemäßen Endoprothese ;

Figur 3 das Verhalten der Endoprothese der Fig. 2 unter ungleichförmiger Belastung, die der ungleichförmigen Belastung von Fig. 1 entspricht ;

Figur 4 einen Querschnitt durch die elastische Zwischenschicht einer anderen Ausführungsform der Erfindung ;

Figur 5 und 6 in ähnlichen Darstellungen wie in Fig. 2, jedoch im Ausschnitt, weitere Ausführungsformen er erfindungsgemäßen Endoprothese.

Die Kniegelenk-Endoprothese besteht aus einem ersten Gelenkteil 1 und einem zweiten Gelenkteil 2, die durch bekannte Einrichtungen 3 gegeneinander festgehalten werden. Der erste Gelenkteil ist an seiner Verankerungsfläche 4, die mit Vorsprüngen 5 versehen sein kann, im Unterschenkelknochen 6 verankert. Der zweite Gelenkteil 2 ist an seinen Verankerungsflächen 7 im Oberschenkelknochen 8 verankert. In dem Bereich, in dem sich die Gelenkteile 1 und 2 berühren, sind am ersten Gelenkteil eine Gleitfläche 9 und am zweiten Gelenkteil eine Gleitfläche 10 und 11 vorgesehen.

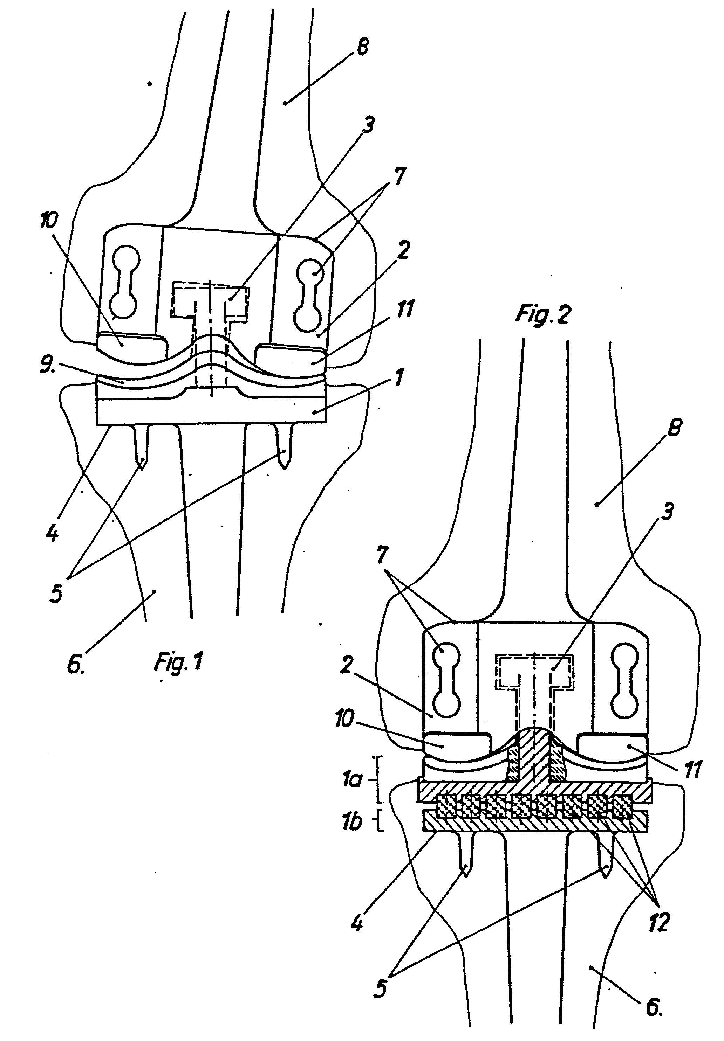

Wird nun, wie in Fig. 1 gezeigt, der Oberschenkelknochen 8 in der Figur nach rechts verschwenkt, so wird die Gleitfläche 10 im in der Figur linken Bereich von der Gleitfläche 9 abgehoben, während die rechte Gleitfläche 11 sehr stark auf die untere Gleitfläche 9 gepreßt wird. Durch diesen starken Druck findet hier erhöhter Abrieb statt. Außerdem treten Probleme bei der Kraftübertragung oder bei Stößen zwischen den beiden Knochen auf, durch die die Verankerung sich im Laufe der Zeit lockern kann.

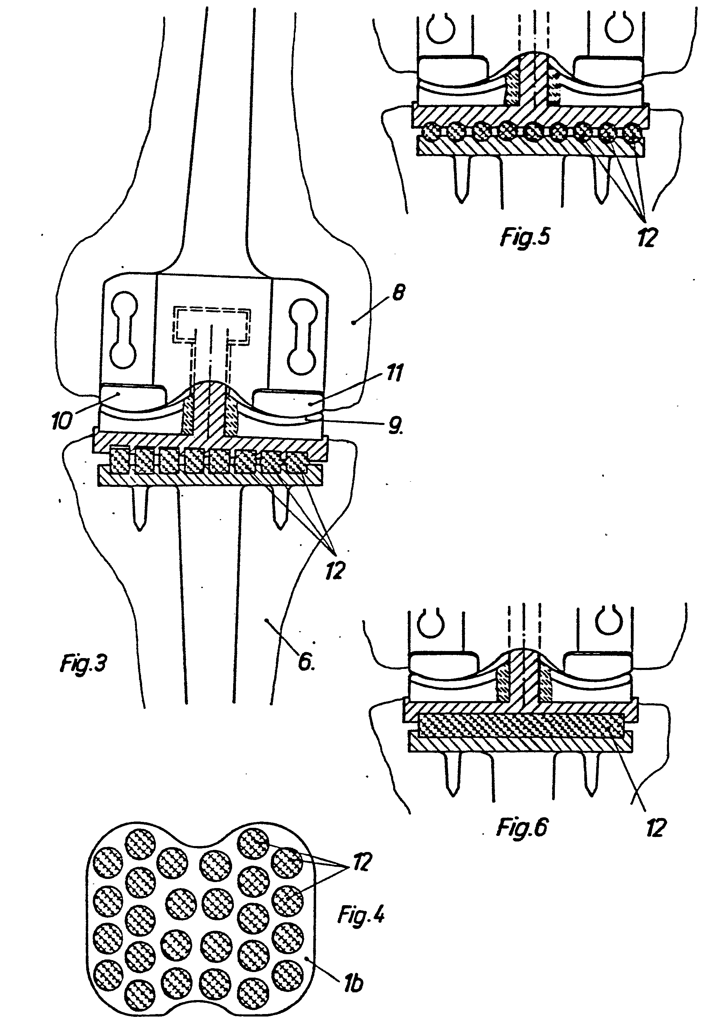

Diese Erscheinung wird dadurch verhindert bzw. gemildert, daß der untere Gelenkteil im wesentlichen aus zwei Teilen besteht, nämlich einem oberen Teil 1a, der die Gleitflächen 9 trägt, und einem unteren Teil 1b, der die Verankerungsfläche 4 und die Verankerungsvorsprünge 5 trägt. Zwischen den beiden Teilen 1a und 1b ist eine Schicht aus elastischem Dämpfungsmaterial in Form einer Vielzahl von elastischen Körpern 12 vorgesehen, die z. B. aus Silikon-Gummi bestehen können. Durch diese elastische Zwischenschicht werden nun Stöße und Kräfte zwischen den Knochen 6 und 8 abgefedert. Außerdem wird, wie aus Fig. 3 ersichtlich ist, bei einer seitlichen Verschwenkung der Knochen im Gelenk zueinander zumindest ein Teil der Kräfte aufgenommen, so daß alle Gleitflächen 9, 10, und 11 miteinander in Berührung bleiben.

Wie aus Fig. 3, 4 und 5 ersichtlich ist, können die elastischen Körper 12 verschiedene Form haben. Sie können z. B. kugelförmig, zylinderförmig (wobei die Zylinderachse senkrecht zur Zwischenschicht stehen kann oder in derselben angeordnet sein kann) oder quaderförmig sein. Gegenüber der Ausführungsform der Fig. 6, wo als elastische Zwischenschicht eine elastische Platte 12 vorgesehen ist, haben die elastischen Körper 12 den Vorteil, daß durch Wegnehmen oder Hinzufügen das elastische Verhalten leicht in gezielter Weise reguliert werden kann.

## Patentansprüche

1. Kniegelenk-Endoprothese mit einem mindestens eine Gelenkgleitfläche und eine Verankerungsfläche bildenden Prothesenteil (1), der aus zwei Einzelteilen (1a, 1b) besteht, von denen einer die Gelenkgleitfläche (9) und der andere die Verankerungsfläche (4) trägt und die unter Zwischenlage von elastischem Dämpfungsmaterial (12) miteinander zur Übertragung von Druck-, Schub- und Torsionskräften verbunden sind, dadurch gekennzeichnet, daß die Verbindung der beiden Einzelteile (1a, 1b) ausschließlich aus dem elastischen Dämpfungsmaterial (12) besteht.

2. Kniegelenk-Endoprothese nach Anspruch 1, dadurch gekennzeichnet, daß das elastische Dämpfungsmaterial (12) plattenförmig ausgebildet ist.

3. Kniegelenk-Endoprothese nach Anspruch 1 oder 2, dadurch gekennzeichnet, daß das elastische Dämpfungsmaterial (12) ringförmig ausgebildet ist.

4. Kniegelenk-Endoprothese nach Anspruch 1, dadurch gekennzeichnet, daß das elastische Dämpfungsmaterial aus einer Vielzahl elastischer Körper (12) besteht, die an einander gegenüberstehenden Flächen der die Gelenkgleitfläche (9) bzw. die Verankerungsfläche (4) bildenden Einzelteile (1a, 1b) befestigt sind.

5. Kniegelenk-Endoprothese nach Anspruch 4, dadurch gekennzeichnet, daß die elastischen Körper (12) zylinder-, kugel- oder quaderförmig sind.

6. Kniegelenk-Endoprothese nach einem der Ansprüche 1 bis 5, dadurch gekennzeichnet, daß die Elastizität des elastischen Dämpfungsmaterials (12) über den von diesem eingenommenen Bereich variiert.

7. Kniegelenk-Endoprothese nach Anspruch 6, dadurch gekennzeichnet, daß elastische Körper (12) unterschiedlicher Größe vorgesehen sind.

8. Kniegelenk-Endoprothese nach einem der Ansprüche 1 bis 7, dadurch gekennzeichnet, daß als elastisches Dämpfungsmaterial (12) Silikon-Gummi verwendet ist.

## Claims

1. A knee-joint endoprosthesis having a prosthesis part (1) which forms at least one joint sliding surface and an anchoring surface and which comprises two individual parts (1a, 1b), one of which carries the joint sliding surface (9) and the other carries the anchoring surface (4) and which are connected to each other, with the interposition of elastic damping material (12), for the transmission of compressive, thrust and torsional forces, characterised in that the connection of the two individual parts (1a, 1b) consists exclusively of the elastic damping material (12).

2. A knee-joint endoprosthesis according to Claim 1, characterised in that the elastic damping material (12) is in the form of a plate.

3. A knee-joint endoprosthesis according to Claim 1 or 2, characterised in that the elastic damping material (12) is of annular shape.

4. A knee-joint endoprosthesis according to Claim 1, characterised in that the elastic damping material (12) consists of a plurality of elastic bodies (12) which are secured to mutually opposing surfaces of the individual parts (1a, 1b) forming the joint sliding surface (9) and the anchoring surface (4) respectively.

5. A knee-joint endoprosthesis according to Claim 4, characterised in that the elastic bodies (12) are of cylindrical, spherical or parallelepipedal shape.

6. A knee-joint endoprosthesis according to one of Claims 1 to 5, characterised in that the elasticity of the elastic damping material (12) varies over the region occupied thereby.

7. A knee-joint endoprosthesis according to Claim 6, characterised in that elastic bodies (12) of different size are provided.

8. A knee-joint endoprosthesis according to one of Claims 1 to 7, characterised in that silicon rubber is used as elastic damping material (12).

## Revendications

1. Endoprothèse d'articulation du genou, comportant un élément de prothèse (1) qui forme au moins une surface de glissement articulaire et une surface d'ancrage et qui se compose de deux parties indépendantes (1a, 1b) dont l'une porte la surface de glissement articulaire (9) et l'autre porte la surface d'ancrage (4) et qui sont assemblées entre elles, avec interposition de matière élastique d'amortissement (12), de manière à transmettre les forces de pression, de cisaillement et de torsion, caractérisée en ce que l'assemblage des deux parties indépendantes (1a, 1b) est assuré exclusivement par la matière élastique d'amortissement (12).

2. Endoprothèse d'articulation du genou selon la revendication 1, caractérisée en ce que la matière élastique d'amortissement (12) est réalisée sous forme de plaque.

3. Endoprothèse d'articulation du genou selon la revendication 1 ou 2, caractérisée en ce que la matière élastique d'amortissement (12) est réalisée sous forme annulaire.

4. Endoprothèse d'articulation du genou selon la revendication 1, caractérisée en ce que la matière élastique d'amortissement est faite d'une multiplicité de corps élastiques (12) qui sont fixés à des surfaces mutuellement opposées des parties indépendantes (1a, 1b) formant respectivement la surface de glissement articulaire (9) et la surface d'ancrage (4).

5. Endoprothèse d'articulation du genou selon la revendication 4, caractérisée en ce que les corps élastiques (12) sont cylindriques, sphériques ou parallélépipédiques.

6. Endoprothèse d'articulation du genou selon l'une quelconque des revendications 1 à 5, caractérisée en ce que l'élasticité de la matière élastique d'amortissement (12) varie sur l'étendue de la région qu'elle occupe.

7. Endoprothèse d'articulation du genou selon la revendication 6, caractérisée en ce qu'il est prévu des corps élastiques (12) de dimensions différentes.

8. Endoprothèse d'articulation du genou selon l'une quelconque des revendications 1 à 7, caractérisée en ce qu'un caoutchouc de silicone est utilisé comme matière élastique d'amortissement (12).

Fig. 1

Fig. 2

Fig.3

Fig.4

Fig.5

Fig.6